Europäisches Patentamt

European Patent Office

Office européen des brevets

(11) Publication number: **0 348 713**
**A1**

# (12) EUROPEAN PATENT APPLICATION

(21) Application number: 89110446.5

(22) Date of filing: 09.06.89

(51) Int. Cl.4: **C07D 295/08** , **A61K 31/495**

Claims for the following Contracting States: ES + GR.

(30) Priority: 28.06.88 IT 2113888

(43) Date of publication of application:
**03.01.90 Bulletin 90/01**

(84) Designated Contracting States:
**AT BE CH DE ES FR GB GR IT LI LU NL SE**

(71) Applicant: **DOTT. FORMENTI S.P.A. INDUSTRIA CHIMICA E FARMACEUTICA**
**Via Correggio, 45**
**I-20149 Milano(IT)**

(72) Inventor: **Passarotti, Carlo**
**Via Correggio, 45**
**I-20149 Milano(IT)**
Inventor: **Valenti, Mauro**
**Via Correggio, 45**
**I-20149 Milano(IT)**
Inventor: **Bandi, Gianluigi**
**Via Correggio, 45**
**I-20149 Milano(IT)**
Inventor: **Fossati, Antonio**
**Via Correggio, 45**
**I-20149 Milano(IT)**

(74) Representative: **Bianchetti, Giuseppe**
**Studio Consulenza Brevettuale Via Rossini, 8**
**I-20122 Milan(IT)**

(54) **(R,S)-3-(4-phenyl-1-piperazinyl)-1,2-propanediol salts.**

(57) (R,S)-3-(4-phenyl-1-piperazinyl)-1,2-propanediol salts with polycarboxylic organic acids such as fumaric, maleic, succinic, tartaric, citric acids and the pharmaceutical compositions containing them have improved pharmaceutical characteristics.

EP 0 348 713 A1

## (R,S)-3-(4-PHENYL-1-PIPERAZINYL)-1,2-PROPANEDIOL SALTS

The present invention relates to (R,S)-3-(4-phenyl-1-piperazinyl)-1,2-propanediol salts with polycarboxylic organic acids.

The invention also relates to pharmaceutical compositions containing said salts as active ingredients.

(R,S)-3-(4-phenyl-1-piperazinyl)-1,2-propanediol, which is also known under the name Dropropizine, is widely used in human therapy, due to its antitussive activity.

Now it has been found that dropropizine salts with polycarboxylic organic acids have unexpected advantages in comparison with the free base or the already described conventional salts, particularly the hydrochloride.

Remarkable advantages are obtained by the therapeutic point of view (improved pharmacokinetics and possibility of new administration routes) as well as by the pharmaceutical technique one (increased stability, higher water-solubility, etc.).

More particularly, the salts according to the present invention allow to overcome the problem caused by undesired colors occuring in the long run in some pharmaceutical formulations based on dropropizine base, particularly by suppositories. Said colors, due to degradation products, are evidenced also when using the hydrochloride, whereas they are completely removed when a salt according to the invention is used.

In the present invention, the term "polycarboxylic organic acids" particularly means di- or tri-carboxylic acid. The salts with dicarboxylic acids selected from the group consisting of maleic, succinic, fumaric and tartaric acids are particularly preferred; dropropizine maleate and fumarate being most preferred.

The salts of the invention can be prepared by direct salification of (R,S)-3-(4-phenyl-1-piperazinyl)-1,2-propanediol, in aqueous or non-aqueous systems.

Water, methanol, ethanol, isopropanol, tetrahydrofuran can be used as salification solvents, ethanol or ethanol/water mixtures being preferred.

The selected acid can be added in subsequent amounts, for example, to a non-aqueous solution of the base. Alternatively, an acid ethanol solution can be added to an ethanol solution of the base. In both cases, the reaction is carried out in stoichiometric amounts of salification agent and dropropizine.

The reaction can be effected at temperatures from 20°C to 40°C, preferably at 25°C.

The salt is recovered by cooling to about 0°C the solution or suspension and subsequently filtering the resulting solid. Alternatively, solvent can be removed off under vacuum and the solid can be recrystallized from an appropriate solvent. Ethanol, methanol and acetone can be used as crystallization solvents, ethanol being preferred.

The obtained salts are conveniently used for the preparation of pharmaceutical compositions to be administered by both the conventional administration routes (oral, rectal or parenteral routes) and the intranasal and transdermic ones, using acceptable non-toxic carriers. Said carriers, being mainly aqueous, low viscosity carriers, tend to increase the absorption rate, similarly to the pharmaceutical formulations using halogenated hydrocarbon carriers as propellers.

Carriers based on gelified matrixes, on O/A or A/O emulsions, used for direct intranasal administration or nebulization, allow to obtain long-lasting effects without significantly affecting the onset quickness. Preferred formulations for the intranasal administration are solutions, creams and gels, whereas preferred formulations for the transdermic administration are gels, creams and medicated patches.

The present invention is illustrated in more detail by the following non-limiting examples, which describe preparation methods as well as pharmaceutical formulations containing the above salts.

### EXAMPLE 1

A solution of 10 g of dropropizine in 200 ml of absolute ethanol was dropped into a solution of 5.8 g of fumaric acid in 50 ml of absolute ethanol, under stirring, at about 25°C, during 1 hour approximately. The resulting solution was cooled under stirring to about 0°C and kept to said temperature for 30 minutes, under stirring.

The obtained salt was filtered, washed with absolute ethanol previously cooled to 5°C, dried in oven under vacuum at 50°C for 2 hours.

14 g of compound were obtained (79.5% yield).

In a similar way, starting from 10 g of dropropizine and 5.8 g of maleic acid, 17 g of the corresponding salt were obtained (96.5% yield).

Chemico-physical characteristics:

DROPROPIZINE FUMARATE

- melting point : 158°C
- solubility : soluble in water and aqueous acid solutions
- UV spectrum (aq. sol.) :
max. absorption at 237 nm ± 2 nm; $E_{1cm}^{1\%}$ = 356 ± 10

| - partition coefficient : | | |
|---|---|---|
| SOLVENTS | P | logP |
| octanol/buffer pH = 1.8 | 0.049 | -1.308 |
| octanol/buffer pH = 7.0 | 1.007 | 0.003 |
| octanol/buffer pH = 9.0 | 1.389 | 0.143 |

DROPROPIZINE MALEATE

- melting point : 95°C (dec.)
- solubility : soluble in water, aqueous acid solutions, methanol and ethanol; almost unsoluble in chloroform.
- UV spectrum (aq. sol.) :
max absorption at 237 nm ± 2 nm; $E_{1cm}^{1\%}$ = 305 ± 10

| - partition coefficient: | | |
|---|---|---|
| SOLVENTS | P | logP |
| octanol/buffer pH = 1.8 | 0.015 | -1.821 |
| octanol/buffer pH = 7.0 | 0.826 | -0.083 |
| octanol/buffer pH = 9.0 | 1.777 | 0.071 |

## EXAMPLE 2

A solution of 10.5 g of citric acid monohydrate in 30 ml of absolute ethanol was added to a solution of 11.8 g of dropropizine in 250 ml of absolute ethanol, at about 25°C. After evaporation of the solvent, a vitreous mass was obtained which could not be crystallized.

After drying in oven at 30°C under vacuum for 4 hours, 22.0 g (98% yield) of the product were obtained.

. In a similar way, starting from 11.8 g dropropizine and 7.5 g of tartaric acid, 18 g of salt (93% yield) as a vitreous not cristallizable mass were obtained.

Chemico-physical characteristics:

DROPROPIZINE CITRATE

- solubility : soluble in water, aqueous acid solutions, methanol and ethanol; almost unsoluble in chloroform
- UV spectrum (aq. sol.) :
max. absorption at 237 nm ± 2 nm; $E_{1cm}^{1\%}$ = 230 ± 10

| - partition coefficient : | | |
|---|---|---|
| SOLVENTS | P | logP |
| octanol/buffer pH = 1.8 | 0.017 | -1.769 |
| octanol/buffer pH = 7.0 | 1.170 | 0.068 |
| octanol/buffer pH = 9.0 | 1.966 | 0.294 |

DROPROPIZINE TARTRATE

- solubility : soluble in water, aqueous acid solutions, methanol and ethanol; almost unsoluble in chloroform;
- UV spectrum (aq. sol.) :

max. absorption at 237 nm ± 2 nm; $E^{1\%}_{1cm} = 305 \pm 10$

| - partition coefficient : | | |
|---|---|---|
| SOLVENTS | P | logP |
| octanol/buffer pH = 1.8 | 0.022 | -1.657 |
| octanol/buffer pH = 7.0 | 1.230 | 0.090 |
| octanol/buffer pH = 9.0 | 1.987 | 0.298 |

| EXAMPLE 3 | |
|---|---|
| Syrup : 100 ml of syrup contain : | |
| Dropropizine citrate | 0.250 - 1.000 g |
| Benzoic acid | 0.090 g |
| Sodium benzoate | 0.050 g |
| Flavors | 0.160 g |
| Saccharose | 72.000 g |
| Depurated water | q.s. to 100.000 ml |

| EXAMPLE 4 | |
|---|---|
| Oral drops : 100 ml of solution contain : | |
| Dropropizine fumarate | 2.500 - 5.000 g |
| Parabens | 0.100 g |
| Flavors | 1.500 g |
| Depurated water | q.s. to 100.000 ml |

| EXAMPLE 5 | |
|---|---|
| Tablets : one tablet contains : | |
| Dropropizine maleate | 10.000 - 50.000 mg |
| Starch | 90.000 mg |
| Lactose | 20.000 mg |
| Talc | 10.000 mg |
| Magnesium stearate | 0.200 mg |

| EXAMPLE 6 | |
|---|---|
| Controlled release tablets : one tablet contains : | |
| Dropropizine maleate | 150.000 mg |
| Hydroxypropylmethylcellulose | 117.000 mg |
| Anhydrous lactose | 80.000 mg |
| Magnesium stearate | 5.000 mg |
| Precipitated silica | 3.000 mg |

| EXAMPLE 7 | |
|---|---|
| Sublingual tablets : one tablet contains : | |
| Dropropizine maleate | 10.000 - 50.000 mg |
| Flavors | 10.000 mg |
| Talc | 10.000 mg |
| Polyvinylpyrrolidone | 4.000 mg |
| Magnesium stearate | 0.200 mg |
| Lactose | |
| Saccharose | q.s. to 150 mg |

## EXAMPLE 8

Chewable tablets : one tablet contains :

| Dropropizine tartrate | 10.000 - 50.000 mg |
|---|---|
| Aspartame | 30.000 mg |
| Flavors | 10.000 mg |
| Magnesium stearate | 10.000 mg |
| Sorbitol | |
| Mannitol | q.s. to 1.000 mg |
| Lactose | |

| EXAMPLE 9 | |
|---|---|
| Cough-drops : one drop contains: | |
| Dropropizine citrate | 10.000 - 50.000 mg |
| Flavors | 20.000 mg |
| Glucose | 1.000 gr |
| Saccharose | 1.500 g |

5

## EXAMPLE 10

Sachets for extemporary dissolution : one sachet contains:

| | |
|---|---|
| Dropropizine tartrate | 25.000 – 50.000 mg |
| Aspartame | 60.000 mg |
| Flavors | 20.000 mg |
| Sorbitol<br>Mannitol ⟶<br>Lactose | q.s. to 1.000 g |

| EXAMPLE 11 | |
|---|---|
| Suppositories : one suppository contains: | |
| Dropropizine fumarate | 0.050 - 0.100 g |
| Guaiacolglyceric ether | 0.200 g |
| Eucalyptol | 0.010 g |
| Semisynthetic solid glycerides | 1.725 g |

| EXAMPLE 12 | |
|---|---|
| Injectable vials : one vial contains: | |
| Dropropizine fumarate | 25.000 - 50.000 mg |
| Apirogen sorbitol | 120.000 mg |
| Water for injectable preparation | q.s. to 3.000 ml |

| EXAMPLE 13 | |
|---|---|
| Intranasal solution : 100 ml of solution contain: | |
| Dropropizine tartrate | 5.000 - 10.000 g |
| Methyl p-hydroxybenzoate | 0.080 g |
| Propyl p-hydroxybenzoate | 0.020 g |
| Glicerin | 20.000 g |
| Depurated water | q.s. to 100.000 ml |

6

| EXAMPLE 14 | |
| --- | --- |
| Intranasal emulsion : 100 g of emulsion contain: | |
| Dropropizine maleate | 5.000 - 10.000 g |
| Methyl p-hydroxybenzoate | 0.080 g |
| Propyl p-hydroxybenzoate | 0.020 g |
| Cetylstearyl alcohol | 18.000 g |
| Polyol esters with fatty acids | 9.000 g |
| Depurated water | q.s. to 100,000 g |

| EXAMPLE 15 | |
| --- | --- |
| Pressurized aerosol : | |
| Dropropizine maleate | 5.000 - 10.000 g |
| Soy lecithin | 0.600 g |
| Ethanol | 5.500 g |
| Freon 113 | 20.000 g |
| Freon 11/12/114 | 70.900 g |

| EXAMPLE 16 | |
| --- | --- |
| Nasal and/or transdermic gel : 100 g of gel contain: | |
| Dropropizine citrate | 5.000 - 10.000 g |
| Methyl p-hydroxybenzoate | 0.080 g |
| Propyl p-hydroxybenzoate | 0.020 g |
| Triethanolamine | 1.100 g |
| Carboxypolymethylene | 1.000 g |
| Propylene glycol | 20.000 g |
| Depurated water | q.s. to 100.000 g |

## EXAMPLE 17

Nasal and/or transdermic ointment : 100 g of ointment contain:

Dropropizine citrate          5.000 – 10.000 g

Methyl p-hydroxybenzoate                0.080 g

Propyl p-hydroxybenzoate                0.020 g

Triethanolamine                         1.250 g

Carboxypolymethylene                    1.000 g

Vaselin oil                             5.000 g

Castor oil                              5.000 g

Liquid paraffin                        10.000 g

Depurated water              q.s. to 100.000 g

| EXAMPLE 18 |  |
| --- | --- |
| Medicated patches : one patch contains: |  |
| Dropropizine maleate | 0.100 - 0.200 g |
| Polyvinylpyrrolidone | 0.100 g |
| Polyvinil Alchol | 0.150 g |
| Glicerin | 1.500 g |
| Depurated water | q.s. to 5.000 g |

| EXAMPLE 19 |  |
| --- | --- |
| Medicated patches : one patch contains: |  |
| Dropropizine fumarate | 0.100 - 0.200 g |
| Precipitated silica | 0.100 g |
| Fluid silicon | 0.850 g |

| EXAMPLE 20 |  |
| --- | --- |
| Medicated patches : one patch contains: |  |
| Dropropizine citrate | 5.000 g |
| Methacrilic copolymers (Eudragit NE 30 D) | 30.000 g |
| Diethylphthalate | 2.000 g |
| Hydroxypropylmethylcellulose | 2.000 g |
| Depurated water | q.s. to 100.000 g |

Claims

1. (R,S)-3-(4-phenyl-1-piperazinyl)-1,2-propanediol salts with polycarboxylic acids.
2. Salts as claimed in claim 1, wherein the polycarboxylic organic acids are di- or tri-carboxylic acids.
3. Salts as claimed in claim 2, wherein di-carboxylic acids are selected from the group consisting of fumaric, maleic, succinic and tartaric acids.
4. (R,S)-3-(4-phenyl-1-piperazinyl)-1,2-propanediol fumarate.

5. (R,S)-3-(4-phenyl-1-piperazinyl)-1,2-propanediol maleate.

6. A process for the preparation of the salts as claimed in claims 1 to 5, in which process stoichiometric amounts of (R,S)-3-(4-phenyl-1-piperazinyl)-1,2-propanediol and polycarboxylic organic acids are reacted in water, water/ethanol, lower alcohols or tetrahydrofuran.

7. Pharmaceutical compositions containing as the active ingredient a salt as claimed in claims 1 to 5 in admixture with a conventional carrier.

8. Pharmaceutical compositions as claimed in claim 7, in form of syrup, drops, tablets, controlled released tablets, chewable tablets, sublingual tablets, cough-drops, sachets, suppositories, vials.

9. Pharmaceutical compositions as claimed in claim 7, for the transdermic or intranasal administration.

10. Pharmaceutical compositions as claimed in claim 9 in form of solutions, emulsions, aerosols, gels, ointments, medicated patches.

11. Salts as claimed in claims 1 to 5 for use as medicaments.

Claims for the following Contracting States: ES, GR

1. A process for the preparation of (R,S)-3-(4-phenyl-1-piperazinyl)-1,2-propanediol salts with polycarboxylic acids, in which process stoichiometric amounts of (R,S)-3-(4-phenyl-1-piperazinyl)-1,2-propanediol and polycarboxylic organic acids are reacted in water, water/ethanol, lower alcohols or tetrahydrofuran.

2. A process as claimed in claim 1, wherein polycarboxylic acids are di- or tri-carboxylic acids.

3. A process as claimed in claim 2, in which di- and tri-carboxylic acids are fumaric, maleic, succinic and tartaric acids.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | GB - A - 2 093 445 (A MENARINI SAS) * Claims * | 1,6-8, 11 | C 07 D 295/08 A 61 K 31/495 |
| A | FR - A - 1 534 651 (M.H.MORREN) * Example 1; table I * | 1,11 | |
| A | CHEMICAL ABSTRACTS, vol. 70, no. 1, January 6, 1969, Columbus, Ohio, USA J.BOURDAIS "Alkylation of piperazines in N,N-dimethyl-formamide" page 330, Abstract-no. 4 039n & Bull.Soc.Chim.Fr.1968,(8), 3246-9 + Chemical Abstracts, Eight Collective Index, Formulas, page 4764F, right column, line 47 | 1 | |

**TECHNICAL FIELDS SEARCHED (Int. Cl.4)**

C 07 D 295/00

The present search report has been drawn up for all claims

| Place of search VIENNA | Date of completion of the search 18-09-1989 | Examiner KÖRBER |
|---|---|---|